# EUROPEAN PATENT SPECIFICATION

(11) **EP 0 261 080 B1**
(45) Date of publication and mention of the grant of the patent: **02.02.1994**
(21) Application number: 87810516.2
(22) Date of filing: 08.09.1987
(51) Int. Cl.: C12N 15/29, C12N 9/08, C12N 9/00, C12N 1/20, C12N 1/18

(54) **Recombinant ligninase**
Rekombinante Ligninase
Ligninase recombinante

(30) Priority: 12.09.1986 US 906853; 11.05.1987 US 48202
(43) Date of publication of application: 23.03.1988
(73) Proprietor: SANDOZ AG, 4002 Basel (CH); SANDOZ-PATENT-GMBH, 79539 Lörrach (DE); SANDOZ-ERFINDUNGEN Verwaltungsgesellschaft m.b.H., 1235 Wien (AT)
(72) Inventor: Farrell, Roberta L., Danvers, MA 01923 (US); Gelep, Paul, Boston, MA 02116 (US); Anilionis, Algis, Pittsford, NY 14534 (US); Javaherian, Kashayar, Lexington, MA 02173 (US); Maione, Theodore E., Concord, MA 01742 (US); Rusche, James R., Framingham,MA 01701 (US); Sadownick, Bruce A., Waltham, MA 02154 (US); Jackson, Jennifer A., Reading, MA 01867 (US)

(56) References cited:
- WO-A-86/02094
- WO-A-87/00550
- BIOCHEM. BIOPHYS. RES. COMMUN., vol. 137, no. 2, 13 June 1986, Academic Press Inc.; Y.Z. ZHANG et al., pp. 649-656#
- NATURE, vol. 326, 02 April 1987; MING TIEN et al., pp. 520-523#
- CHEMICAL ABSTRACTS, vol. 103, no. 23, 09 December 1985, Columbus, OH (US); U. RAEDER et al., p. 362, no. 192667n#
- CHEMICAL ABSTRACTS, vol. 104, no. 11, 17 March 1986, Columbus, OH (US); R. HAYLOCK et al., p. 329, no. 84795q#
- ENZYME MICROB. TECHNOL., vol. 8, January 1986, Butterworth & Co. Ltd.; T.K. KIRK et al., pp. 27-32#
- PROCEEDINGS OF THE NATL. ACADEMY OF SCIENCES USA, vol. 81, April 1984; M. TIEN et al., pp. 2280-2284#
- NATURE, vol. 328, 20 August 1987; M. TIEN et al., p. 742#

## Description

This invention relates to lignin modifying and degrading enzymes. Lignin is a complex polymer formed by free radical polymerization of substituted cinnamyl alcohol precursors, which constitutes 15-35% of dry wood weight. During pulping processes, cellulosic fibers must be liberated from their encasing lignin matrix so that they can associate with one another, yielding strength in the final product. This polymer separation can be accomplished by removal of lignin as in chemical pulps, or by maintaining the lignin as in high yield mechanical pulps. During the bleaching process, lignin is removed and the resulting pulp is brightened.

Lignin is highly resistant to biological attack. No organism has been demonstrated to grow on lignin as the sole carbon source. The complex lignin polymer, however, is completely degraded by pure cultures of various higher order fungi. The most extensive physiological investigations of lignin biodegradation by white-rot fungi have been conducted with a single member of the family Corticraceae, Phanerochaete chrysosporium Burds.

Under defined laboratory conditions, fungal lignin degradation is not observed during approximately the first 3 days of culture. Subsequently, the culture becomes starved for carbon or nitrogen, and lignin degradation is first observed at day 4 or 5 of culture and is maximal at 6 days. The induction of lignin degradation in response to carbon and nitrogen starvation indicates that fungal lignin metabolism is a secondary metabolic event.

Fungal lignin degradation currently is commercially impractical for several reasons. The rate of lignin degradation is unacceptably slow since ligninolytic activity is induced only as a result of secondary metabolism. Therefore, a period of initial growth followed by starvation is required. Furthermore, fungi metabolize cellulosic fibers as their primary food source, resulting in reduced pulp yield and an inferior pulp product.

PCT application WO 87/00550 describes a lignin-degrading enzyme preparation designated rLDM™ 6 which is derived from P. chrysosporium (rLDM™ is the trademark of the Repligen Corporation, Cambridge, MA, for lignin degrading and modifying enzymes). This preparation is substantially pure and hence also substantially free of other rLDM™ and degrading native proteases. The enzyme consists of a protein component (apoprotein) bound to a heme unit which was identified as being the oxidized form (Fe3⁺) of protoheme IX. This rLDM™ 6 preparation possesses desirable properties for use in pulping wood and treating effluent. The rLDM™ exhibits peroxidase activity which has been characterized by the critical property of being able to catalyze the oxidation of veratryl alcohol to veratrylaldehyde in the presence of hydrogen peroxide. The following physical parameters of the rLDM™ were also disclosed:
(1) molecular weight as determined by SDS-PAGE;
(2) amino acid composition;
(3) heme content;
(4) homology by antibody reactivity;
(5) specificity of activity against lignin model substrates; and
(6) elution from a FPLC column at specified sodium acetate molarities.

Though the preparation of rLDM™ 6 can be carried out as described in WO 87/00550 it could be expected that more efficient production of rLDM™ 6 would be realized by genetic engineering methods if DNA encoding rLDM™ 6 protein could be cloned and expressed in a suitable host. However, it was unpredictable whether there could be expression of activity of a P. chrysosporium gene in a heterologous host since this had never been achieved previously, and the prior art provides no suggestions as to how such a cloning and expression could be achieved.

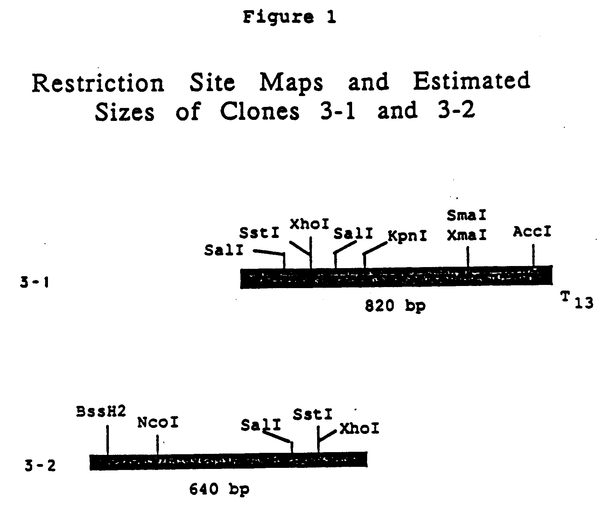

Y.Z. Zhang et al. in Biochem. Biophys. Res. Commun. Vol. 137(2) (13th June 1986), pages 649-656 reported an attempt to clone the same protein as in the present invention. They prepared three very short probes based on two short protein sequences obtained from several tryptic peptide fractions which were impure, both sequences (as seen in light of the present invention) falling within the first 100 amino acids of the 344 amino acid protein as now established by the applicants. One of the sequenced lengths does not even exactly correspond to this protein. A clone bank was prepared and screened with the three probes. Three clones hybridised with one of the three probes and one clone (pCLG5), with a 1.48 Kb insert, hybridized with all three probes. For this reason, it was concluded but not demonstrated that the protein "corresponds to the main ligninase protein H8 described above" and all hybridised clones were concluded to be "indeed ligninase cDNA clones". However, no expression or confirmation of ligninase activity was demonstrated nor were any of the clones sequenced. The publication acknowledges that anti-bodies raised against H8 (by others) can bind to different ligninases and this leads to the logical further acknowledgment concerning the uncertainty as to how the genes are in fact constructed and whether common genes may be involved. There is a high potential to clone only a portion of a sought-for DNA and to obtain undesired contributions from the messengers for different but similar proteins. And indeed wrong conclusions were drawn in the article of Zhang et al. when comparing the information therein with the disclosure of the present invention. In particular, on page 655 of the article it is indicated that the identified H8 clone pCLG5 has no Sal I site. As can be seen from Figure 1, there is, however, such a site on both clones 3-1 and 3-2 and in fact this site falls in the overlapping area between the two clones the inventors pieced together to achieve success. Moreover, Zhang et al. indicated an EcoRI site while no such site is found in applicants coding sequence.

In fact, H.A. de Boer, Y.Z. Zhang, C. Collins and C. Adinarayana Reddy first came to the conclusion that the protein they obtained was identical with rLDM™6 (GENE 60 (1987), 93-102) but they had to revise their first opinion (GENE 69 (1988), 369) and came to the conclusion that their cDNA encodes for a different ligninase.

The present invention provides recombinant rLDM™6 ligninase enzyme, by means of the cloning of a DNA sequence encoding the ligninase apoprotein and its expression in suitable host cells and by reconsitituting the recombinant ligninase apoprotein with protoheme IX.

Thus the invention provides the DNA sequence encoding the rLDM™ 6 protein of native origin from P. chrysosporium, which sequence is shown in Table 1. From the DNA sequence, the amino acid sequence of the protein has been deduced as shown in the same Table.

Cloning of the DNA sequence coding for rLDM™ 6 protein was initiated by preparing sufficient quantities of RNA from a fermentation of Phanerochaete chrysosporium. Enhanced quantities of RNA were prepared from a UV-induced mutant of ATCC 24725, designated SC26, NRRL 15978.

After copying the mRNA with reverse transcriptase, a gene bank comprising DNA sequences coding for genes involved in secondary metabolism including lignin-modifying or -degrading enzymes was constructed. Antibody reactive against rLDM™ 6 was used to probe the bank. Cross-reactive clones were detected. Phage DNA was prepared from isolates of the clones, using standard procedures. This DNA was then inserted into suitable vectors for subsequent expression of the rLDM™ 6 protein into competent hosts.

A wide variety of modified recombinant rLDM™ 6 proteins which are capable, when reconstituted with the heme, of substantially the same rLDM™ 6 enzymatic activity are also included within the scope of the invention. Structural modifications can either be constructed by known procedures or be of a natural origin since rLDM™ proteins expressed from other native allelic genes most probably exist.

rLDM™ 6 proteins which are modified by genetic engineering have been also shown to be capable of ligninase activity when reconstituted with a heme. For example, proteins comprising the mature rLDM™ 6 sequence extended by one or more amino acids at its normal N-terminus have been produced as described below. Protein comprising thed mature sequence extended at its normal C-terminus by additional amino acids may also be produced as indicated herein by causing the expression of expression plasmids in an amber host.

Within the definition of the modified rLDM™ 6 proteins are also subfragments of the original protein expressed from DNA sequences which hybridize to the discovered DNA sequence for original rLDM™ 6 protein.

When reconstituted with the heme the original recombinant rLDM™ 6 protein or the modified rLDM™ 6 proteins can be used in the same manner as the natural enzyme to degrade or modify lignin; and have activity on kraft lignin, such as found in unbleached kraft pulps, on milled-wood lignin, such as found in mechanically-derived pulp and on lignin model compounds.

The gene for the rLDM™ 6 protein can also be isolated from genomic DNA, a so-called genomic clone, by using partial sequences from the cDNA of rLDM™ 6 protein as a probe. The techniques are well known in the art; see Maniatis, T., Fritsch, E.F. and Sambrook, J. Molecular Cloning: A Laboratory Manual, Cold Spring Harbor Laboratory, NY, 1982. The genomic clone thus obtained can be inserted into suitable vectors for various hosts.

Additionally to the DNA sequence as disclosed in Table I, modified DNA sequences including native allelic genes and DNA sequences modified by genetic engineering and encoding rLDM™ 6 proteins capable of rLDM™ 6 enzymatic activity, when reconstituted, are also within the scope of the invention.

Allelic genes representing one or more modifications in the DNA sequence discovered as coding for rLDM™ 6 proteins may be obtained, for example, from natural sources by probing suitable cDNA or genomic DNA banks derived from P. chrysosporium or similar wood-degrading fungi with one or more appropriate lengths of tagged DNA excised from the DNA coding for the mature sequence of rLDM™ 6 as diclosed herein, having at least about 20 nucleotides to about 300 nucleotides.

Modified sequences encoding the modified proteins above described also may be prepared directly from the cDNA or genomic DNA sequence disclosed herein as coding for rLDM™ 6 proteins by employing established known techniques such as site specific mutagenesis, loop-out procedures and other known methods for substituting nucleotides.

Having the nucleotide sequence of the original rLDM™ 6 protein and of the modified rLDM™ 6 proteins, those skilled in the art can readily appreciate the identity of other equivalent nucleotide sequences coding for rLDM™ 6 proteins. As is well known, different nucleotide sequences can code for the same protein because of the redundancy of the genetic code, i.e. most amino acids are encoded by more than one nucleotide triplet (codon). Thus, the scope of the subject invention includes not only the specific nucleotide sequence disclosed herein, but also all equivalent nucleotide sequences coding for molecules which are capable of substantially the same rLDM™ 6 ligninase activity when reconstituted with the heme.

Since the nucleotide sequence coding for rLDM™ 6 protein is herein disclosed it also can be synthesized by a "gene machine" by procedures well known in the art.

Hence, in a broader aspect of the invention, nucleotide sequences provided in accordance with this invention include those cDNA and/or genomic DNA sequences which code for protein having ligninase activity and which hybridize to the sequence coding for mature rLDM™ 6 protein as disclosed in Table 1 hereof under stringent hybridizing conditions (e.g. 60°C in 2.5x saline citrate buffer). However, as will also be appreciated, a cDNA and/or genomic DNA coding sequence of native origin may be substantially modified or reconstructed to produce very dissimilar DNA sequences coding for the same similar protein, typically for purposes of enhancing expression in heterological or non-native host systems. Hence, in its broadest aspect, the invention provides isolated recombinant or synthetic DNA sequences coding for rLDM™ 6 and modified rLDM™ 6 proteins which are capable of the ligninase activity characteristic of rLDM™ 6.

A wide variety of methods are available for introducing the rLDM™ 6 gene into the microorganism host under conditions which allow for stable maintenance and expression of the gene. One can provide for DNA constructs which include the transcriptional and translational regulatory signals for expression of the gene, the gene under their regulatory control and a DNA sequence homologous with a sequence in the host organism, whereby integration will occur, and/or a replication system which is functional in the host, whereby integration or stable maintenance will occur.

The essentially pure nucleotide sequences coding for rLDM™ 6 or for biologically active subfragments of rLDM™ 6 can be ligated into appropriate restriction enzyme sites in an expression cloning vector. If necessary, sites can be added to nucleotide sequences using linker molecules. (See for example, Norris, K.E. et al. [1979] Gene 7:355-362.) The ligated DNA can then be used to transform competent prokaryotic or eukaryotic cells.

rLDM™ 6 proteins can be expressed in Saccharomyces cerevisiae using plasmids containing the inducible galactose promoter from this organism (Broach, J.R., Li, Y., Wu, L.C. and Jayaram, M. in Experimental Manipulation of Gene Expression [1983] p. 83, ed. M. Inouye, Academic Press). These plasmids are called YEp51 and YEp52 (Broach, J.R. et al., 1983, supra) and contain the E. coli origin of replication, the gene for β -lactamase, the yeast LEU2 gene, the 2 µm origin of replication and the 2 µm circle REP3 locus. Recombinant gene expression is driven by the yeast GAL10 gene promoter.

Other yeast promoters such as alcohol dehydrogenase (Bennetzen, J.L. and Hall, B.D. [1982] J. Biol. Chem. 257:3018; Ammerer, G. in Methods in Enzymology [1983] Vol. 101, p. 192) phosphoglycerate kinase (Derynck, R., Hitzemann, R.A., Gray, P.W., Goeddel, D.V., in Experimental Manipulation of Gene Expression [1983] p. 247, ed. M. Inouye, Academic Press), triose phosphate isomerase (Alber, T. and Kawasaki, G. [1982] J. Molec and Applied Genet. 1: 419 - 434), or enolase (Innes, M.A. et al. [1985] Science 226:21) can be used in a similar manner.

The rLDM™ 6 gene can be expressed by the filamentous fungus Aspergillus since this fungus is related to P. chrysosporium. A suitable host vector which can be used for gene expression in Aspergillus is the vector described in Buxton, F.P., Gwynne, D.I. and Davis, R.W. Gene [1985] 37:207.214.

rLDM™ 6 protein can be expressed in mammalian cells using for example the Okayama-Berg cloning system (Okayama, H. and Berg, P. [1983] Molec. and Cell. Biol. 3:280-289), the vaccinia virus expression system (Moss, B. [1985] Immunology Today 6:243; Chakrabarti, S., Brechling, K., Moss, B. [1985] Molec. and Cell. Biol. 5:3403) or the vectors derived from murine retroviruses (Mulligan R.C. in Experimental Manipulation of Gene Expression [1983] p. 155, ed. M. Inouye, Academic Press).

Since the ligninase protein must be reconstituted with a heme in order to exhibit a peroxidase activity on lignin and lignin-derived substrates, the invention also provides a method for reconstituting an active heminic protein which comprises the step of reacting the apoprotein with a heme in the presence of a redox system.

A recombinant ligninase enzyme was further obtained according to this method by using the recombinant ligninase protein of the invention. This recombinant ligninase enzyme exhibits the same ligninase activity as the purified natural rLDM™ 6. The enzyme produced by recombinant DNA technique is indicated to degrade/modify lignin without significantly attacking cellulose or hemicellulose. Like the natural enzyme, the rLDM™ 6 enzyme is immediately active and requires no metabolic induction as is necessitated by use of natural fungal isolates.

The terms "peroxidase protein", "ligninase apo-protein" or "ligninase protein" denotes herein a protein which has not been reconstituted with a heme and which is capable of having a peroxidase and/or ligninase activity when reconstituted with a heme.

The term "protoheme IX" denotes the oxidized Fe³⁺ form of protoheme IX.

The term "ligninase enzyme" refers to a ligninase protein which has been reconstituted with protoheme IX.

### Culture Deposits

The following deposits of cultures disclosed in this application have been made in the Agricultural Research Culture Collection (NRRL), Northern Regional Research Center, U.S. Department of Agriculture, 1815 North University Street, Peoria, Illinois, USA, under the provisions of the Budapest Treaty for the Deposit of Microorganisms.

| Culture | Repository No. | Deposit Date |
|---|---|---|
| E.coli(pLn105) | NRRL B-18156 | December 31, 1986 |
| E.coli(pLn106) | NRRL B-18157 | December 31, 1986 |
| E.coli(pBSR3) | NRRL B-18068 | May 1, 1986 |
| E.coli K12 JM105 | NRRL B-18067 | May 1, 1986 |
| E.coli(pREV2.2) | NRRL B-18091 | July 30, 1986 |
| S.cererisiae(pLn1001) | NRRL Y-18163 | January 14, 1987 |

### Description of the Drawings

- FIGURE 1:: Restriction Site Maps and Estimated Sizes of Clones 3-1 and 3-2
- FIGURE 2:: MATURE rLDM™ 6 N-Terminal Sequence
- FIGURE 3:: Alignment of Mature rLDM™ 6 N-Terminal Sequence with Early Sequence from Clone 3-2
- FIGURE 4:: Time Course of Activity Recovery-Reconstituted rLDM™ 6

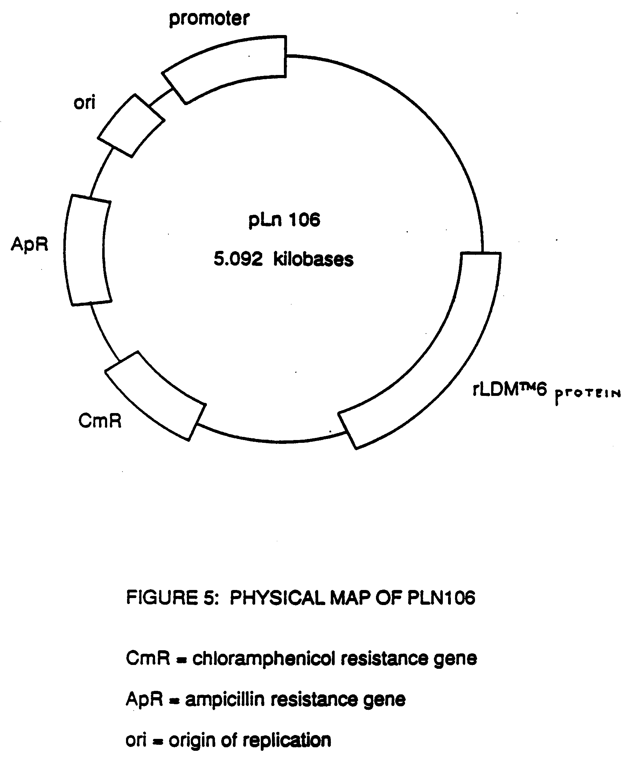

- FIGURE 5:: Physical Map of pLn106
- FIGURE 6:: Schematic of pLn106 Construction
- FIGURE 7:: Schematic of pREV2.2 and of Multiple Cloning Site
- FIGURE 8:: Schematic for construction of pLn105
- FIGURE 9:: Schematic for construction of pTPR
- FIGURE 10:: Schematic for construction of pLn1001.

Following are examples which illustrate procedures for practicing the invention. All percentages are by weight and all solvent mixture proportions are by volume unless otherwise noted.

### Techniques and Materials

Most of the techniques which are used to extract mRNA, prepare cDNA libraries, transform cells, construct vectors and the like are widely practiced in the art, and most practitioners are familiar with the standard resource materials which describe specific conditions and procedures. These procedures are for example, described in Maniatis, T., Fritsch, E.F. and Sambrook, J., (1982) Molecular Cloning: A Laboratory Manual, Cold Spring Harbor Laboratory, New York. Thus, it is within the skill of those in the genetic engineering art to extract DNA from microbial cells, perform restriction enzyme digestions, electrophorese DNA fragments, tail and anneal plasmid and insert DNA, ligate DNA, transform cells, prepare plasmid DNA, electrophorese proteins, and sequence DNA.

All restriction endonucleases used herein were purchased from Bethesda Research Laboratories, Gaithersburg, MD or New England Biolabs, Beverly, MA, and were used according to the directions of the supplier.

DNA sequence determination was carried out using procedures described by Maxam and Gilbert (Maxam, A. and Gilbert, W. [1977] Proc. Natl. Acad. Sci. USA 74:560) and Heidecker et al. (Heidecker, G., Messing, J. and Gronenborn, B. [1980] Gene 10:69).

Agarose gel electrophoresis was carried out using a 2X Tris-acetate gel buffer (80 mM Tris-HCl, pH 8.0; 40 mM sodium acetate; 36 mM NaCl; 2 mM Na₂ EDTA) in the gel and 1X buffer for the run. Analytical gels were routinely run as "submarine gels" in a horizontal gel box. DNA bands were visualized by ethidium bromide (EtBr) post-staining (0.5 mg/ml in 1X gel buffer) and use of a UV transilluminator Model TM-36 from UltraViolet Products, Inc., San Gabriel, CA.

Extraction of DNA from preparative agarose gels was initiated by visualization of the positions of EtBr-stained bands of a single gel lane. Gel slices containing DNA fragments of interest were diced manually and passed through a 20 g needle with 1½-2 volumes DNA gel extraction buffer (0.5 M ammonium acetate, 10 mM EDTA, 10 mM Mg acetate, 0.1% SDS). An equal volume of phenol saturated with 1 mM ammonium acetate, 10 mM EDTA was added and extraction carried out in Eppendorf tubes on a rotary shaker at 23°C overnight. The tubes were then placed on ice for 30 min prior to separation of the aqueous phase by microcentrifugation.

Extraction of the aqueous phase with the saturated phenol solution was repeated 3-4 times, followed by chloroform extraction and ethanol precipitation. Routine recovery of DNA fragments smaller than 15 kb was about 40%.

### Example 1 - Culture Source of RNA

P.chrysosporium NRRL 15978 was grown on a nitrogen-limited trace element medium supplemented with glucose and buffered at pH 4.5.

Ligninase activity in the fermentation media was measured periodically by standard means determining the rate of oxidation of veratryl alcohol to veratrylaldehyde.

Isolation and purification of the novel rLDM™ from the extracellular fluid in the fermentation was accomplished by ultrafiltration and FPLC using an anion exchange column.

### (A) Growth conditions

Cultures of NRRL 15978 were grown in 125 ml Erlenmeyer flasks as stationary growths, in a disk fermenter, in 1 liter or 2 liter shaking Erlenmeyer flasks, or in a 20 liter fermenter. The first 2 types of cultures were grown in the following media, designated nitrogen-limited BIII/glucose medium:
1.08 x 10⁻³ M ammonium tartrate, 1.47 x 10⁻² M KH₂PO₄, 2.03 x 10⁻³ M MgSO₄·7H₂O, 6.8 x 10⁻⁴ M CaCl₂·2H₂O, 2.96 x 10⁻⁶ M thiamine·HCl and 10 ml·L⁻¹ of a trace element solution. The trace element solution contains 7.8 x 10⁻³ M nitrilo-acetic acid, 1.2 x 10⁻² M MgSO₄·7H₂O, 1.7 x 10⁻² M NaCl, 3.59 x 10⁻⁴ M FeSO₄·7H₂O, 7.75 x 10⁻⁴ M CoCl₂, 9.0 x 10⁻⁴ M CaCl₂, 3.48 x 10⁻⁴ M ZnSO₄·7H₂O, 4 x 10⁻⁵ M CuSO₄·5H₂O, 2.1 x 10⁻⁵ M AlK(SO₄)₂·12H₂O, 1.6 x 10⁻⁴ M H₃BO₃, 4.1 x 10⁻⁵ M NaMoO₄·2H₂O and 2.9 x 10⁻³ M MnSO₄·H₂O.

The medium was supplemented with 10% (by wt/liter) of glucose and buffered with 10.8 mM ammonium tartrate, pH 4.5 with 7x trace metals (basal level +6x) and 0.04 M veratryl alcohol. The shake flask cultures and 20 liter fermenter were in the same media but with the addition of 0.1% Tween 80.

The 125 ml stationary Erlenmeyer flasks containing 10 ml of culture medium were inoculated with 0.5 ml of the mycelial inoculum and flushed on days 0, 3, and 6 with O₂.

The disk fermentors contained 2 l culture medium. Inoculation was by addition of 100 ml of the mycelial inoculum. Within 1 day after inoculation, the scored disks were covered by a thin mycelial mat. The rotating biological contactors (RBCs) were flushed continuously through ports in the cover with O₂ at 100 ml per min.

The 1 liter or 2 liter shaking Erlenmeyer flasks, containing 300 or 600 ml culture medium, respectively, were inoculated with 50 or 100 ml of the mycelial inoculum. The flasks were shaken in a New Brunswick (New Brunswick Scientific, Edison, NJ) incubator at 250 rpm at 39°C. Cultures were flushed on days 0, 2, 4 and 6 with O₂.

The 20 liter Virtis fermenter (Series 43) (The VirTis Co., Inc., Gardiner, NY) containing 10-14 liters of culture medium, was inoculated with the non-homogenized fungus from a 7-day-old ligninase positive 1 liter (300 ml) shaking Erlenmeyer flask. The impeller blades of the fermenter rotated at 300 rpm. Oxygen was introduced by diffusion through approximately 50 feet of Corning silastic 0.058 inch medical tubing (Corning Glass Works, Corning, NY). Oxygen flow equalled 300 ml/min.

### (B) Ligninase assay

Ligninase activity in the flasks or RBCs was measured periodically by determining the rate of oxidation of veratryl alcohol to veratrylaldehyde (abbreviated as VAO for veratryl alcohol oxidation). Reaction mixtures contained 275 µl of extracellular fluid, or approximately 0.5 µg of purified enzyme, 2 mM veratryl alcohol, 0.4 mM H₂O₂ and either 20 or 100 mM sodium tartrate, pH 2.9 in a final volume of 0.5 ml. The reactions were started by H₂O₂ addition and were monitored at 310 nm. Protein was determined according to Bradford, M.M., (1976) Anal. Biochem. 72:248-254, using bovine serum albumin (Sigma Chemical Co., St. Louis, MO) as standard or by using the 409 nm absorbance of a protein solution and calculating protein amount from the extinction coefficient of ligninase.

### (C) Purification of ligninases

The extracellular fluid, prepared as described above, was prefiltered through a Millipore (Millipore Corporation, Bedford, MA) 0.45 um filter and concentrated 20-fold by ultrafiltration through an Amicon (Danvers, MA) YM10 filter (henceforth, this concentrate is called Ligninolytic Mixture, LM™), dialyzed against 10 mM sodium acetate, pH 6.0, and filtered through 0.45 µm filters (Acrodisc Low Protein Binding, Gelman, Ann Arbor, MI). HPLC (high performance liquid chromatography) was performed with the filtered LM™ on a Gilson (Gilson Co., Inc., Worthington, OH) system equipped with an LDC/Milton Roy (Milton Roy Co., Rochester, NY) fixed 410 nm wavelength detector as well as a Gilson variable wavelength detector using a Pharmacia (Piscataway, NJ) FPLC Mono Q anion exchange column to purify the ligninases according to their elution from the column. The mobile phase consisted of a gradient from 10 mM to 1 M sodium acetate, pH 6.0, and in typical preparations was applied over a 40 min period at a flow rate of 2 ml per min with constant monitoring at 410 nm and 280 nm. Each ligninase of interest eluted at a unique sodium acetate molarity from the column (Kirk et al. [1986] Enzyme Microb. Technol. 8:27-32).

### (D) Polyacrylamide gel electrophoresis (PAGE)

The ligninases produced by P. chrysosporium, NRRL 15978, were analyzed by SDS-polyacrylamide slab gel electrophoresis according to Laemmli, U.K. (1970) Nature 227:680-685. Native slab gels were run according to the procedures of Ornstein, L. (1964) Ann. N.Y. Acad. Sci. 121:321-349; and Davis, B.J. (1964) Ann. N.Y. Acad. Sci. 121:404-427. After electrophoretic separation, the proteins were visualized by coomassie blue staining, or Western blot analysis using standard procedures.

### Example 2 - RNA Preparation

Total RNA was prepared from veratryl alcohol oxidizing fermentation broths of P. chrysosporium. The harvested and washed mycelia are freezed in liquid nitrogen and crushed with mortar and pestle for lysis. The cell powder was then suspended in guanidium thiocyanate denaturing solution and centrifuged in CsCl. The RNA band was isolated and passed through an oligo(dT)-cellulose column to obtain the polyA⁺ mRNA, as described in the Maniatis Manual.

### Example 3 - cDNA Preparation

Ten micrograms of polyA⁺ mRNA, as obtained in Example 2, was primed with oligodT₁₂₋₁₈ and copied with reverse transcriptase essentially as described by Gubler and Hoffman (Gubler, U. and Hoffman, B.J. [1983] Gene 25:263-269).

The cDNA was cloned into the well-known and commercially-available vector lambda-gtll (Young, R.A. and Davis, R.W. [1983] Proc. Natl. Acad. Sci. USA 80:1194-1198; available from Vector Cloning Systems, San Diego, CA). Double stranded cDNA was methylated with EcoRI methylase enzyme (New England Biolabs, Beverly, MA) and S-adenosyl-methionine as recommended by the supplier and ligated with a mixture of three EcoRI linkers (GGAATTCC, CGGAATTCCG and CCGGAATTCCGG). These linkers ensure that fusions in all three translational reading frames can be obtained.

The EcoRI linked cDNA was cleaved with EcoRI endonucleases and a fraction from 600 to 2,000 base pairs was isolated from a 1% agarose gel. This purified EcoRI cleaved cDNA was ligated with EcoRI-cut, phosphatase-treated lambda-gtll DNA, packed into phage and used to infect E. coli Y1088, ATCC 37195.

### Example 4 - Antibody Screening

Rabbit anti-rLDM™ antibody reactive against HPLC-purified P. chrysosporium LDM™ 6 was used to probe the bank derived from the 600-2,000 bp cDNA, as described by Young et al. (Young, R.A. and Davis, R.W. [1983] Proc. Nat. Acad. Sci. 80:1194-1198). Cross-reactive clones were detected.

Phage DNA was prepared from isolates of each of the cross-reactive clones by standard procedures (see, for example, Maniatis, T. et al.) and digested with EcoRI to reveal the size of the cDNA insert. The insert size of the clone designated 3-1 as estimated is 820 bp. Four other clones also contained similar inserts of approximately 650 bp, of which clone 3-2 is representative.

Figure 1 shows the restriction maps of the DNA inserts of clones 3-1 and 3-2. Clone 3-1 contains the expected oligodT tract (used to prime the cDNA synthesis) contiguous with an EcoRI linker sequence.

### Example 5 - N-Terminal Characterization of rLDM™ 6 protein

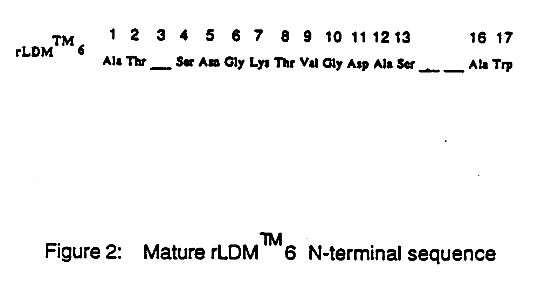

rLDM™ 6 was purified from the extracellular growth medium of P. chrysosporium from mutant SC26 by ion-exchange HPLC as described by Kirk et al. (Kirk, T.K., Croan, S., Tien, M., Murtagh, K.E. and Farrell, R.L. [1986] Enzyme Microb. Technol. 8:27-32). This isolate was subjected to N-terminal amino acid analysis by HPLC (Hunkapiller, M. and Hood, L. [1983] Methods in Enzymol. 91:227, 486). The N-terminal results are given in Figure 2.

Cysteine is not detected in this system and thus the third, 14th and 15th amino acids were registered as blanks.

### Example 6 - Characterization of the Sequence encoding the ligninase rLDM™ 6 protein

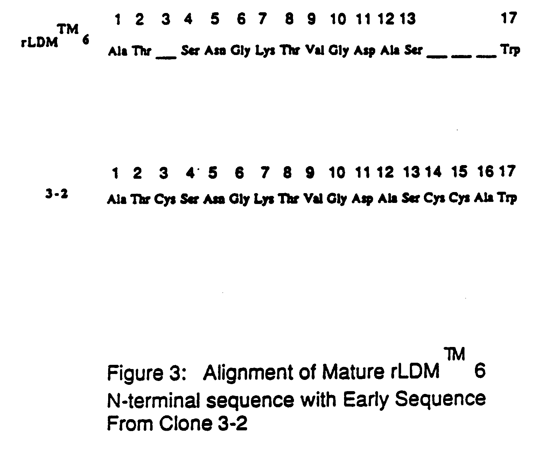

Analysis of DNA sequence information from the presumed 5' end of the clone 3-2 showed a sequence consistent with the experimentally determined mature protein N-terminus, as shown in Figure 3. Since a 145 bp region of clones 3-1 and 3-2 was shown to be a perfect match, a full length cDNA clone was reconstructed by splicing together clones 3-2 and 3-1 at their common SstI sites, yielding an EcoRI-SstI-EcoRI fragment of approximately 1280 bp. The sequence of this fragment is given in Table I.

This EcoRI-SstI-EcoRI fragment (herein referred to as RI fragment) was cloned in the EcoRI-digested plasmid pREV2.2 (described in US patent application Serial No. 892680). The resulting plasmid is pLn102.

This represents a very non-standard cloning procedure by independently finding the predicted N-terminal and C-terminal halves of the protein, respectively.

When the RI fragment is appropriately inserted into a suitable expression vector, e.g., a plasmid, and the vector is used to transform a non-amber suppressed bacterium, e.g., E. coli JM105, the host bacterium, upon cultivation, expresses the mature rLDM™ 6 protein whose amino acid sequence, as shown in Table 1. extends from the 1-position Ala to a C-terminus of the amino acid sequence at the Ala (position 344) just prior to the stop signal at location 345. When an amber suppressed host such as E. coli JM103 is transformed by the vector, the C-terminus of the amino acid sequence shown in Table I is Gly at amino acid position 361, which is located prior to the stronger stop signal at location 362. The amino acid sequence of the mature rLDM™ 6 apo-protein predicted from the cDNA sequence consists of 344 residues, extending from the 1-position Ala to the 344-position Ala.

### Example 7 - Construction of Plasmid pBSR3

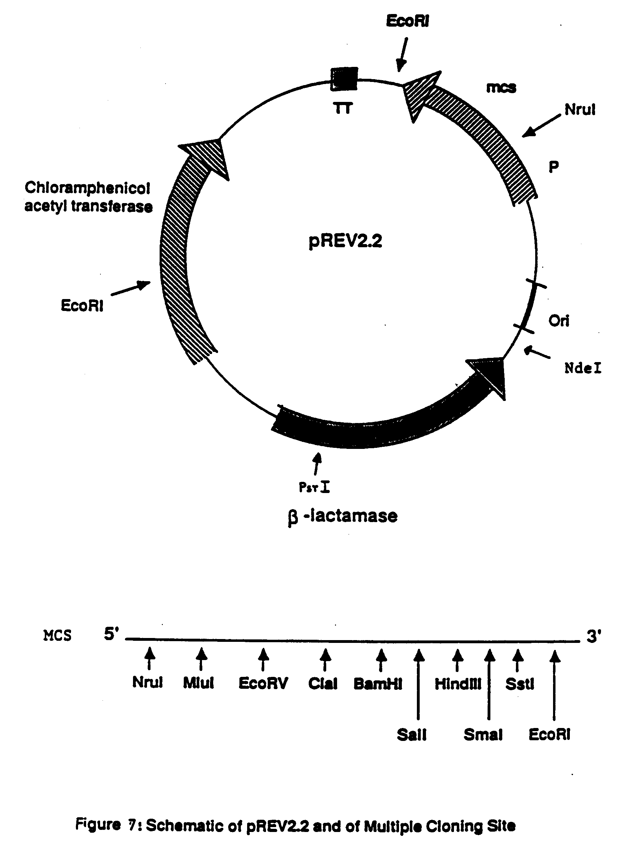

Since a portion of the codon for the Ala at the beginning of the mature rLDM™ 6 apoprotein sequence includes a portion of a BssHII site (GCGCGC), the balance of said site preceding the Ala codon and including all of the CGC codon for Arg at a minus 1-position, a BssHII-SstI-EcoRI DNA fragment containing all of the coding strand information for rLDM™ 6 apoprotein was excised from the cloning plasmid pLn102 (Example 6) by first cutting the same with the restriction endonuclease BssHII and filling in the resulting 5' overhang by treatment with the Klenow fragment of DNA polymerase, hence fully reestablishing the DNA for encoding the amino acids Ala and Arg. The resulting linearized and treated plasmid DNA was then digested with the endonuclease EcoRI to free the desired fragment. This fragment after gel isolation was used to form the expression plasmid pBSR3 as a protein fusion system with the plasmid pREV2.2 shown in Fig. 7. As shown in Fig. 7, the plasmid pREV2.2 includes a multiple cloning site (MCS) downstream from a promoter (P). Restriction sites within the multiple cloning site are shown in the linearized MCS segment in Fig. 7. To prepare pBSR3, the plasmid pREV2.2 was totally digested with the restriction endonucleases EcoRI and EcoRV and the resulting linearized plasmid was ligated with the above-obtained and modified BssHII-SstI-EcoRI fragment by standard ligating procedure. Hence, the sequence coding for the mature protein beginning with GCC (positions 91, 92 and 93 in Table I) in pBSR3 is preceded at its 5' or upstream end by CGC coding for Arg, and this in turn is immediately preceded by 87 bp coding for 29 amino acids (contributed by the MCS section) which, in turn, are immediately preceded by ATG coding for Met. Thus, pBSR3 codes for an amino acid sequence which has the mature sequence of rLDM™ 6 apoprotein preceded at its ordinary N-terminus by a section of 31 amino acids which has a Met as its N-terminus. The expression of pBSR3 in a non-amber host is described in Example 8 below.

### Example 8 - Fermentation and extraction of Recombinant rLDM™ 6 protein from E. coli (pBSR3)

A 20 liter fermenter of E. coli(pBSR3), NRRL B-18068, was grown in Modified 2% Yeast Extract Medium without oxygen control for 24-32 hr. The composition of the medium is as follows:

| Ingredient | Amount |
|---|---|
| yeast extract | 20.0 gm/liter |
| casein peptone | 20.0 gm/liter |
| casamino acids | 20.0 gm/liter |
| KH₂PO₄ | 2.0 gm/liter |
| K₂HPO₄ | 2.0 gm/liter |
| Na₂HPO₄·7H₂O | 2.0 gm/liter |
| chloramphenicol | 100.0 gm/liter |

When aliquots of the harvested cells were lysed and analyzed by SDS-PAGE and visualized with coomassie blue protein stain, a protein band with apparent molecular weight of approximately 41,000 becomes more prominent compared to non-transformed cell controls. That this band represented rLDM™ 6 apoprotein was verified by Western blot analysis using antibody reactive toward native rLDM™ 6 (Towbin, H., Staehelin, T. and Gordon, J. [1979] Proc. Natl. Acad. Sci. USA 76:4350-4354) which revealed the major immunoreactive protein also to correspond to this molecular weight. This protein is not glycosylated, which distinguishes it from the native (P. chrysosporium-produced) protein.

When cells were lysed by sequential lysozyme and bead mill treatment, recombinant rLDM™ 6 apoprotein was detected only in the pellet fraction after centrifugation.

For subsequent solubilization of the recombinant rLDM™ 6 protein the pellet fraction is treated with a 4-8M urea solution or with about 6M guanidine solution. Use of a 8M urea solution solubilizes 90 to 95% of the rLDM™ 6 protein, while a 4M urea solution only solubilizes 50% of the protein.

When a 4M urea solution (4M urea in TE buffer pH 8 50 mM Tris-HCl 1 mM EDTA) is added to the lysed-culture pellet, 50% of the protein still remains in the pellet which is further isolated from the supernatant. Subsequent addition of a 8M urea solution allows the entire or almost entire solubilization of the remaining 50%. Hence, more than 95% of the protein can be solubilized by the above procedure.

It has also been found that addition of about 10 mM dithiothreitol enhances the efficiency of solubilization in a 4M urea solution to almost the level of an 8M urea solution.

### Example 9 - Preparation of Expression Plasmid pLn104

Plasmid pBSR3 (Example 7) was the basis for a standard loop-out experiment to form a modified pBSR3 in which all but one of the codons (the CGC coding for Arg prior to the GCC for the 1-position Ala) in the nucleotide sequence between the initiating ATG and GCC for the 1-position Ala were excised (looped-out). To effect this result, the plasmid pBSR3 was treated with the restriction endonucleases EcoRI and NdeI and the resulting fragment containing the ligninase coding sequence was gel isolated. Another portion of pBSR3 was linearized by treatment with the restriction endonuclease PstI and gel isolated. The two thus isolated segments were then combined with a single stranded 40 base nucleotide designed to effect the desired loop-out and having the sequence TTAATGAGGAGTCCCTTATGCGCGCCACCTGTTCCAACGG. The resulting heteroduplex was treated with DNA polymerase and the resulting system worked upon in a conventional fashion to isolate the desired expression plasmid, designated pLn104. Upon tranforming E. coli JM105 with pLn104 and culturing the successful transformants, there was expressed a 345 amino acid ligninase protein having the amino acid sequence of mature rLDM™ 6 protein preceded at its normal N-terminal by Arg.

### Example 10 - Construction of expression plasmid pLn105

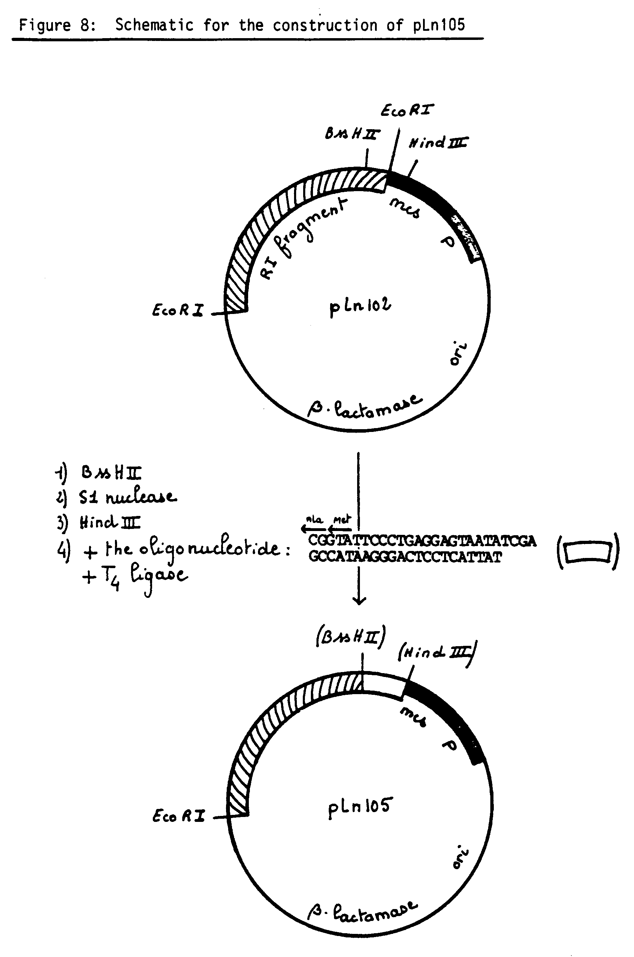

The plasmid pLn105 was constructed as an operon fusion using the E. coli promoter available from pREV2.2 and a linkage downstream therefrom of an oligonucleotide containing a standard non-coding ribosomal binding site followed by an initiator ATG codon which is to be placed immediately prior to the GCC which codes for the 1-position Ala of the mature protein. Hence, pLn105 is designed to code for the amino acid sequence of the mature protein preceded at its N-terminus by Met. To summarize from Figure 8, the plasmid pLn105 was prepared by digestion of the cloning plasmid pLn102 (Example 6) (formed by simple ligation of EcoRI cut pREV2.2 with the approximately 1280 bp EcoRI-SstI-EcoRI fragment) with the restriction endonuclease BssHII. The resulting linearized plasmid was then digested with S1 nuclease by standard procedures to remove the 5' overhang created by the BssHII digestion. The resulting fragment was then digested with the restriction endonuclease HindIII which cuts in the multiple cloning site section of the plasmid pREV2.2. The resulting large fragment was then ligated by standard ligation procedures with the oligonucleotide 
AGCTATAATGAGGAGTCCCTTATGGC
TATTACTCCTCAGGGAATACCG
which contains a non-coding ribosomal binding site GGAGTCCCTT attached to initiating methionine (ATG) codon and GC portion of the alanine codon (in order to reestablish the GCC coding for the 1-Ala of the mature protein). pLn105 was expressed in E. coli host JM103, NRRL B-39403. The N-terminus expresses as methionine-alanine-threonine, etc. as per Table 1. Methionine was not detected in the majority of rLDM™ 6 protein isolated.

### Example 11 - Construction of Plasmid pLn106

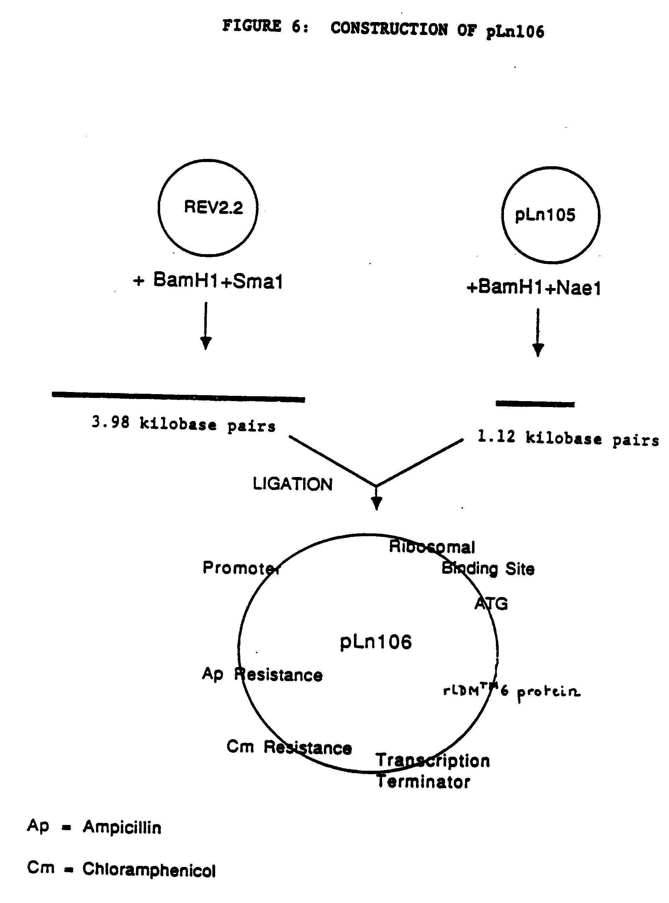

Plasmid pLn106 was constructed as indicated in the schematic given in Pig. 6. To summarize from Fig. 6, the plasmid pREV2.2 was digested with the restriction endonucleases BamHI and SmaI which cut in the area of the MCS of REV2.2. The resulting large fragment of about 3,980 bp of DNA was isolated from the digestion fragments. The plasmid pLn105 was digested with the restriction endonucleases BamHI (which cuts in the MCS section originating in pREV2.2) and with NaeI (which cuts between the nucleodides in positions 1170 and 1171), and the resulting fragment of about 1,118 bp was isolated from the digestion fragments. The above isolated fragments of about 3,980 and 1,118 bp were then ligated together using standard ligation procedures. The resulting plasmid pLn106 contains the genes for chloramphenicol and ampicillin resistance, an origin of replication, an E. coli promoter, a non-coding ribosomal binding site of the sequence GGAGTCCCTT, the ATG initiating codon and the DNA sequence starting with position 91, GCC... to position 1170,... GTGGCC, as given in Table 1. This DNA sequence includes the sequence encoding rLDM™ 6 protein starting at position 91 and extending to position 1122 (ending GGTGCT).

### Example 12 - Expression of recombinant rLDM™ 6 apoprotein in E. coli (pLn106)

The plasmid pLn106 was transformed by standard techniques into E. coli, SG 20251 (NRRL 15918) and a 20-liter fermentation was grown by culturing as in Example 7 to induce expression of rLDM™ 6 apoprotein. The harvested cells were lysed by sequential lysozyme and bead mill treatment. After centrifugation, recombinant rLDM™ 6 apoprotein was detected mainly in the pellet fraction. Recombinant rLDM™ 6 apoprotein was isolated from the insoluble pellet fraction, as in Example 8. The protein solution was purified to 30% by extraction in 4 M urea, 50 mM sodium acetate, pH 6, 10 mM dithiothreitol (DTT) and resuspended with a POLYTRON (Brinkman Instruments, Westbury , NY) for 30 sec. to 3 min. The supernatant extract was then separated from the pellet by appropriate centrifugation. The protein solution was applied on a DEAE-SEPHAROSE anion exchange column (Pharmacia, Piscataway, NJ) in Buffer A (4 M urea, 50 mM sodium acetate, pH 6, and 5 mM DTT). A gradient of 0 to 0.5-1.0 M NaCl was run in Buffer A. Fractions were collected and analysed by SDS-polyacrylamide slab gel electrophoresis (see Example 1). After eletrophoresis separation the proteins were visualized by coomassie blue staining. The apparent size of rLDM™ 6 produced from this procedure from E. coli strain SG20251 (NRRL B-15918) is approximately 39.5 kilodaltons (kd). rLDM™ 6 produced from pLn106 in E. coli is not glycosylated, which distinguishes it from the native (P. chrysosporium-produced) protein.

The fractions collected from the DEAE-Sepharose column were also checked for their immunoreactivity with anti-rLDM™ 6 ligninase antibody. The most strongly immunoreactive fractions were pooled (25 ml) and applied to a sizing column, S-300 (400 ml) Sephacryl (Pharmacia) in 4 M urea, 50 mM KH₂PO₄, pH7.0, 4 mM DTT. Again immunoreactivity of fractions (10 ml each) from the sizing column was checked and the most strongly reactive were pooled. When examined by SDS-PAGE this pooled fraction appeared to be approximately 20% rLDM™ 6 as judged by scanning the coomassie blue staining pattern.

The first 8 N-terminal amino acids of rLDM™ 6 so prepared have been determined to be Ala Thr --- Ser Asn Gly Lys Thr, where the blank in this system is generally a cysteine residue. Therefore the initiating ATG codon encoding methionine was not found to be present under these conditions of fermentation and production. The N-terminal methionine may be present in other preparations of rLDM™ 6 depending upon E. coli strain, expression level, and method of purification.

### Example 13 - Reconstitution of the recombinant rLDM™ 6 ligninase

Heretofore no successful heme reconstitution of ligninases from P. chrysosporium or of recombinant ligninases has been demonstrated.

In order to realize good activity of rLDM™ 6, the starting material can be frozen cells or freshly harvested cells. The protein solution is prepared as described in Example 10 . The urea extraction mix may be stirred at room temperature for up to 6 hr, at which time the extract is centrifuged from 30 min to 15 hr, depending upon the gravitational force of the centrifugation. The anion exchange column is run immediately and the pooled fractions are put into the reconstitution protocol within 24 hr, unless the pooled rLDM™ 6-containing fractions are held at -20°C, where they can be stored longer before reconstitution.

Successful reconstitution, with activation of rLDM™ 6 to at least a specific activity of 26 units veratrylaldehyde produced from veratryl alcohol/min/mg rLDM™ 6, has been achieved with rLDM™ 6 preparations which range in purity from 5-100%. These rLDM™ 6 preparations were made from cells expressing either clone pLn105 or pLn106 (see Examples 10, 11 and 12). These preparations can be in a protein concentration of 0.1 to 1 mg/ml in their elution buffer from the anion exchange column which is typically 0.1 to 0.3 M NaCl. The protein preparation used for reconstituting the enzyme can also be obtained after elution on a sizing column of the fractions collected from the DEAE sepharase column, as described in Example 12. rLDM™ 6 protein isolated from the sizing column is estimated at 20% purity.

Before operating the reconstitution procedure, the protein solution obtained from the DEAE-sepharase column or from the sizing column must be first dialysed to remove urea from the protein solution. Because of the presence of DTT in the buffered protein solution, the protein is used for reconstitution under its reduced form.

One procedure used is as follows: The pooled fraction from the sizing column at 3 mg/ml total protein concentration, with recombinant rLDM™ 6 representing about 20% of total protein, is in a buffer of 4M urea, 50 mM KH₂PO₄, pH 7.0, 4 mM DTT. For removing urea this is dialysed against a 200-fold volume excess of buffer of 50 mM Tris-Cl, pH 8.0, 1 mM DTT, 20% (v/v) glycerol overnight at 4°C. After dialysis, a four-fold molar excess of protoheme IX (Sigma) dissolved freshly in 0.1 N KOH is added to this protein solution. This is then dialyzed against 50 mM Tris-Cl, pH 8.0, 1 mM reduced glutathione, 100 µM oxidized glutathione, overnight at 4°C. Finally, the sample is dialyzed against an aqueous solution containing 10 mM sodium acetate, pH 6.0, overnight at 4°C. Modifications of this reconstitution procedure have also been demonstrated to be effective. One modification consists of dialysing the rLDM™ 6 protein solution against a buffer solution containing the reduced and oxidized glutathiones before protoheme addition. One experiment is as follows:

The protein solution obtained for the sizing column as above described is dialysed against a 200-fold volume excess of buffer comprising 50 mM Tris-Cl, pH 8.0, 1 mM reduced glutathione, 100 µM oxidized glutathione, overnight at 4°C. Then a 4-fold molar excess of protoheme is added to the dialysed protein solution and finally the solution obtained is further dialysed against an aqueous solution containing 10 mM sodium acetate at a pH of 6.0 overnight at 4°C.

Reconstitution is indicated by the presence of a Soret absorption band at 409 nm.

Broadly, a reconstitution protocol comprises
(a ) adding a 1-fold to 10-fold molar excess of a heme to the solution of an apoprotein;
(b) dialyzing the solution obtained in (a) against a dialysis solution comprising 0.1 to 5 mM of a reduced sulfhydryl redox system, and 10 to 500 µM of an oxidized sulfhydryl redox system, at a pH of 5 to 10.

Preferably the dialysis solution may also contain 5 to 30 % (V/V) glycerol, more preferably 20 % glycerol.

A modified reconstitution protocol is characterized broadly by
(a) dialyzing a buffered solution of an apoprotein against a dialysis solution comprising 1 to 5 mM of a reduced sulfhydryl redox system, and 10 µM to 500 µM of an oxidized sulfhydryl redox system at a pH of 5 to 10;
(b) adding a 1-fold to 10-fold molar excess of a heme to a solution of the apoprotein obtained in (a).

In order to remove the excess of the heme the solution obtained in (b) of the two reconstitution processes may be further dialysed against an agueous solution preferably at a pH of 4 to 8.

If a reconstitution procedure does not give a product with good specific veratryl alcohol oxidation activity there are several procedures, called reactivation procedures, which will result in rLDM™ 6 material with high specific activity. One such reactivation procedure is to dilute the initially reconstituted material to 0.1 mg/ml to 0.5 mg/ml rLDM™ 6 in 10-200 mM potassium phosphate or equivalent buffer, pH 5-7. Add 4-fold molar excess (over rLDM™ 6 protein) of a protoheme IX. Incubate for 1-24 hr at 4°C to 37°C and repeat the dialysis step in 10-200 mM potassium phosphate or equivalent buffer, pH 5-7 from 4-24 hr at 4°C to 37°C. Alternatively, the initially reconstituted material, with no dilution, has a 4-fold molar excess of protoheme IX added followed by passage through a Sephadex G-50 column in 10-200 mM potassium phosphate or equivalent buffer, pH 5-7. The resulting eluant containing rLDM™ 6 material has superior specific activity to the initial material.

### Example 14 - Activity of recombinant rLDM™ 6 ligninase

Reconstituted recombinant ligninase rLDM™ 6 enzyme obtained as described in Example 13 has been demonstrated to be active in lignin model compound assays and with lignin.

The lignin model compound assays that have been used are (1) the veratryl alcohol oxidation (VAO) assay, (2) the 1,2,4,5-tetramethoxybenzene (TMB) assay, and (3) the adlerol assay.

The VAO assay tests for oxidation of a C_{α} aromatic alcohol to an aldehyde and is as described in Example 1.

The TMB assay tests for demethoxylation. The products of the ligninase reaction, consuming 1 mole hydrogen peroxide per 1 mole of substrate, are 2 moles methanol and 2,5-methoxybenzoquinone. Reaction conditions are 2 to 5 µg of purified enzyme, 20 mM sodium tartrate, pH 2.5, 64 µM hydrogen peroxide, and 1 mM 1,2,4,5-tetramethoxybenzene. The reaction is monitored at 450 nm, indicative of the yellow aryl cation radical intermediate species.

The adlerol assay tests for cleavage of the C_{α} - C_{β} bond and resulting oxidation to a C_{α} aldehyde. The reaction conditions and monitoring are identical to the VAO assay, except that alderol replaces veratryl alcohol.

The lignin assay is carried out by measuring the rate of hydrogen peroxide consumption with time using a YSI model 25 Oxidase meter with a YSI 2510 Oxidase probe (Yellow Springs Instrument Co., Inc., Yellow Springs, OH). The reaction conditions are identical to the VAO assay but with 40 µM hydrogen peroxide and veratryl alcohol replaced by either milled wood lignin or kraft lignin at 20 to 250 µg/ml.

Chart 1 gives typical specific activities, measured in the VAO and TMB assays of rLDM™ 6 protein, expressed from E. coli NRRL B-18157 (non-amber host) (see Example 12), and reconstituted as described in Example 13 compared to specific activities of a pool of native ligninases wherein P. chrysosporium rLDM™ 6 is predominant. Specific activity with adlerol for rLDM™ 6 enzyme was comparable to that of P. chrysosporium rLDM™ 6 enzyme.

### Chart I

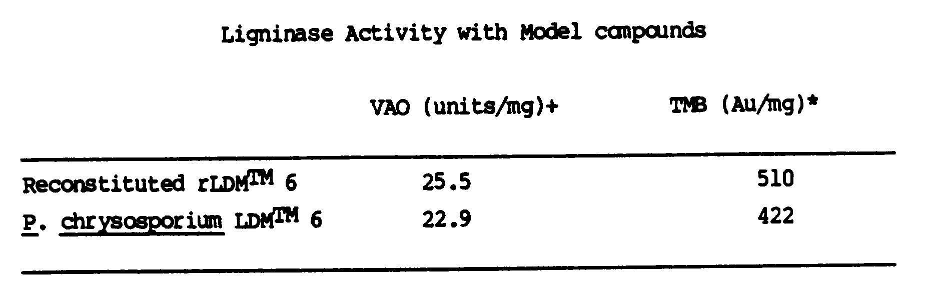

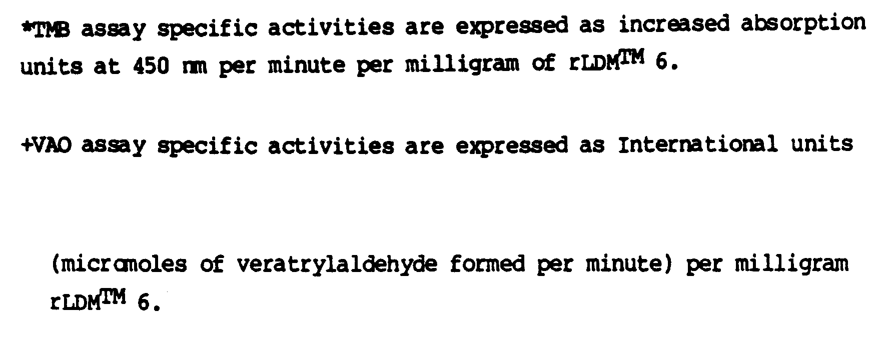

The specific activities of reconstituted recombinant ligninase with lignins, demonstrated after a 5 min addition, are 76.9 units/mg rLDM™ 6 on Indulin AT (kraft lignin) and 66.0 units/mg rLDM™ 6 on loblolly pine milled wood lignin. (A unit represents consumption of 1 nanomole of hydrogen peroxide per min).

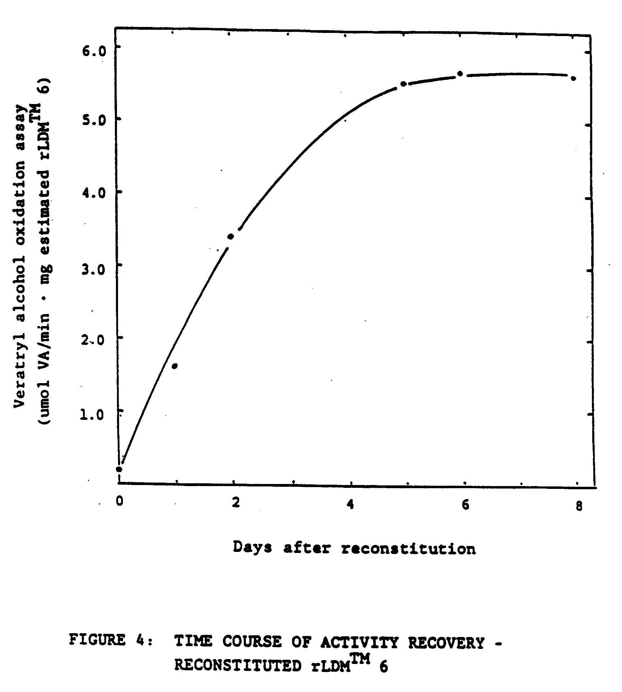

The last step in reconstitution is dialysis against 10 mM sodium acetate, pH. 6.0. The activity of the reconstituted rLDM™ 6 increases upon storage after this last dialysis. Figure 4 shows the increase in VAO activity with reconstituted rLDM™ 6 over an 8-day period with storage at 4°C. Day 0 represents the day the sample is taken out of the 10 mM sodium acetate, pH 6.0 dialysis. The VAO assays are run as previously described. The activity over the 8-day period has increased by 10-fold. After day 5 of storage, the activity does not further increase but remains very stable. P. chrysosporium ligninases do not show any increase in activity after dialysis and upon storage at 4°C.

This increase in activity with time of reconstituted rLDM™ 6 has been observed also in the assays with the lignins in the peroxide consumption assays and the TMB assay. An increase in activity after isolation has not been observed with the native P. chrysosporium material.

The activity of 7-day stored rLDM™ 6 calculated from peroxide consumption, is 76.9 unit/mg rLDM™ 6, where a unit represents consumption of 1 nanomole hydrogen peroxide per minute versus 39.1 unit/mg rLDM™ 6 of 6-day stored enzyme. The increase in activity may be temperature dependent. Chart 2 shows the data from an experiment done with the same reconstituted rLDM™ 6 material used and represented in Figure 4. Reconstitution of the desired protein products of the other foregoing examples, results in ligninase protein having a similar VAO activity, except that the protein from pBSR3 (Example 7) exhibited a lower order of such activity.

### Chart 2

| Increased Activity of rLDM™ 6 Temperature Dependent | | |
|---|---|---|
| Day | Storage Temperature | VAO activity Units/mg rLDM™ 6 (all assays at 37°C) |
| 5 | starting material | 5.55 |
| 6 | 4°C | 5.70 |
| | | 5.65 |
| | 23°C | 7.25 |
| | | 7-05 |

The duplicates left at room temperature increased in activity over the duplicates left at 4°C by 26 %.

### Example 15 - Construction of plasmid pLn1001 and Expression of recombinant rLDM™ 6 protein in S. cerevisiae

pLn1001 was constructed by ligation of a linearized yeast - E. coli shuttle vector YEp6 (Struhl, K. et al. Proc. Nat. Acad. Sci. 76 : 1035-1039 (1979)) with an insert containing the nucleotide sequence of rLDM™ 6 initiating with position 91 and ending with position 1172 (see Table I) between the yeast triose phosphate isomerase (TPI) transcription promoter and terminator; this insert also comprises, between the TPI promoter and the rLDM™ 6 encoding sequence a 66 bp additional oligonucleotide containing the invertase signal sequence (INV-ss) Carlson, M., Taussig, R., Kustu, S. and Bostein, D., Hol. Biol. (1983) 439-447) so that excretion of rLDM™ 6 protein will be allowed when expressed in S. cerevisiae.

The insert is constructed as separate 5' and 3' portions which are joined in YEp6 as a second step.

### A) Construction of plasmid pTPR from which the TPI promoter will be further isolated

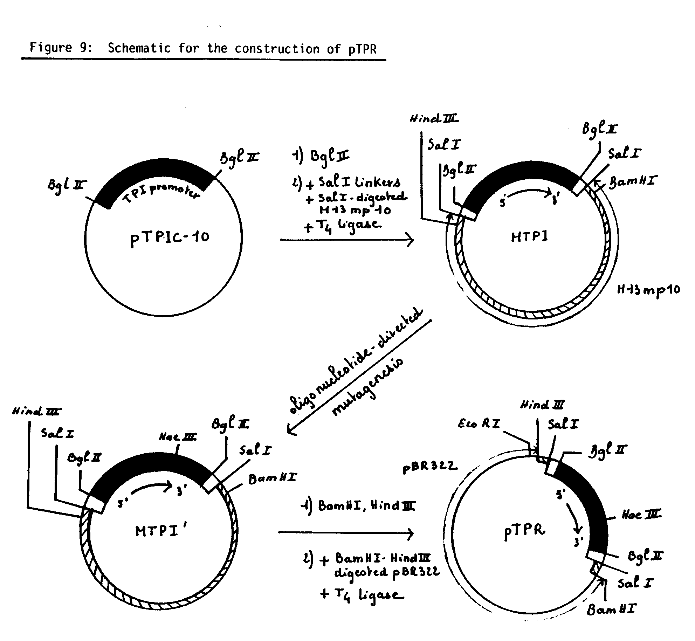

To summarize from Fig. 9, the 1200 bp BglII - BglII fragment of pTPIC-10 (Alber, T. and Kawasaki, G. J. Molec. and Applied Gen. 1:419-434 [1982]) containing TPI promoter and flanked by SalI linkers is ligated with M13 mp10 vector (Messing, J. and Viera, J. Gene (1982) 19:269-276) previously digested by SalI, in order to obtain MTPI vector in which the HindIII and BamHI sites of M13 mp10 are adjacent respectively to the 5' and 3' ends of the TPI promoter.

Then a oligonucleotide-directed mutagenesis (Vlasuk, G.P. and Inouye, S. Experimental Manipulation of gene expression (ed. H. Inouye) Academic Press : 291-303) and (Itakura, K., Rossi, J.J. and Wallace, R.B. (1984) Ann. Rev. Biochem. 53 : 323-356) is effected so that a HaeIII site is created immedially following the codon of the N-terminal methionine of the TPI.

The BamHI-HindIII fragment containing the TPI promoter is removed from MTPI. Plasmid pTPR is the product of the ligation of this fragment with pBR322 previously digested by BamHI and HindIII.

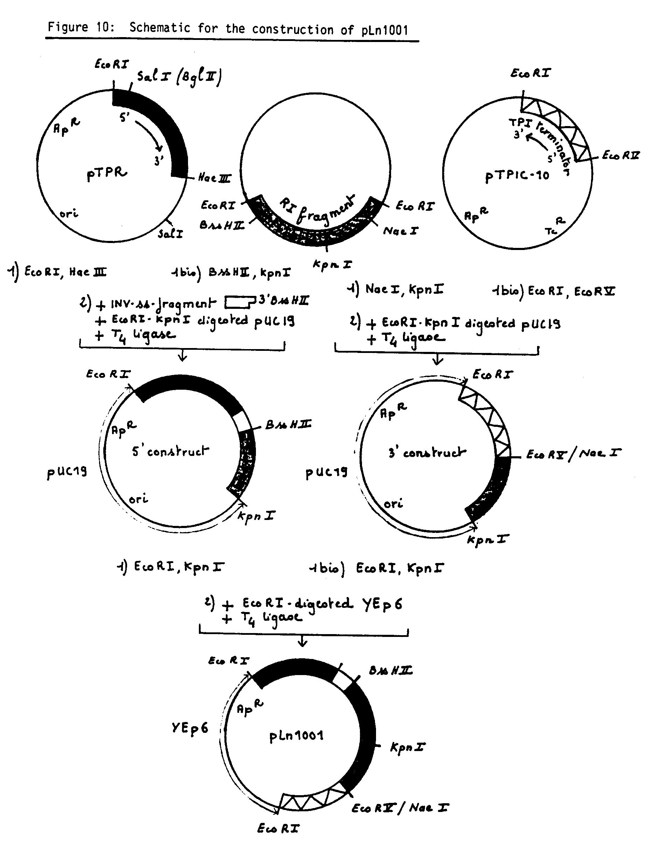

### B) Construction of the 5'-portion (as indicated in Figure 10)

The TPI promoter is gel-isolated from plasmid pTPR as an appropriate 1000 bp EcoRI-HaeIII fragment.

A plasmid containing the RI fragment is linearized with BssHII and KpnI (KpnI cuts the rLDM™ 6 encoding sequence between the nucleotides in positions 728 and 729) A 637 bp fragment is generated containing the 5' end of the rLDM™ 6 encoding sequence which is isolated from an agarose gel.

A 66 bp fragment which sequence is 
CTCTTTTGCAAGCTTTCCTTTTCCTTTTGGCTGGTTTTGCAGCAAAATATCTGCATCAATG
GAGAAAACGTTCGAAAGGAAAAGGAAAACCGACCAAAACGTCGGTTTTATAGACGTAGTTGCGCGC
and which contains the invertase signal sequence is designed with a blunt 5' end and a BssHII overlap at the 3' end and is synthesized as four oligonucleotides; 1A is the 5' 40 bases of the coding strand, 1B the 3' 22 bases of the coding strand, 2A the 5' 45 bases of the non-coding strand and 2B the 3' 21 bases of the non-coding strand. To prevent concatemer formation, only 1B and 2B were kinased. The four oligonucleotides were annealed and ligated and the resulting INV ss fragment gel isolated.

The approximate 1000 bp EcoRI-HaeIII fragment, the 637 bp BssHII-KpnI fragment and the 66 kp fragment are ligated together into EcoRI-KpnI-digested pUC19 (Yanisch-Perron, C. Viera, J. and Messing, J. (1985)).

### C) Construction of the 3'portion (as indicated in Figure 10)

The approximate 700 bp EcoRI-EcoRV fragment from plasmid pTPIC-10 which contains the TPI transcription terminator is gel isolated.

The 444 bp NaeI-KpnI fragment of rLDM™ 6 encoding sequence is also gel-isolated.

The 700 bp and 444 bp fragments are ligated into EcoRI-KpnI-digested pUC19.

### D) Construction of pLn1001 (as indicated in Figure 10)

The KpnI-EcoRI fragment containing the 5' portion and the KpnI-EcoRI fragment containing the 3' portion are gel isolated respectively from the plasmids constructed in B) and C), and ligated with YEp6 digested with EcoRI.

### E) Expression of the recombinant ligninase apoprotein H8 in S.cerevissae

The resulting plasmid, pLn1001 is transformed into the host S. cerevisiae DBY747 (University of California, Berkeley, Yeast Genetic Stock Center). DBY747 (pLn1001) is inoculated in minimal medium supplemented with leucine, uracil, tryptophan, methionine, casamino acids (Difco, Detroit, MI) and glucose and incubated 18 hr at 30°C with vigorous shaking. Alternatively, the host can he grown in synthetic medium containing 0.67% yeast nitrogen base, 2% glucose and 0.5% casamino acids (Difco). Cells are harvested and lysed by bead milling. From the insoluble cell pellet, rLDM™ 6 protein, of apparent molecular size 41 kd, is detected by immunological reaction with polyclonal antibodies made to P. chrysosporium rLDM™ 6.

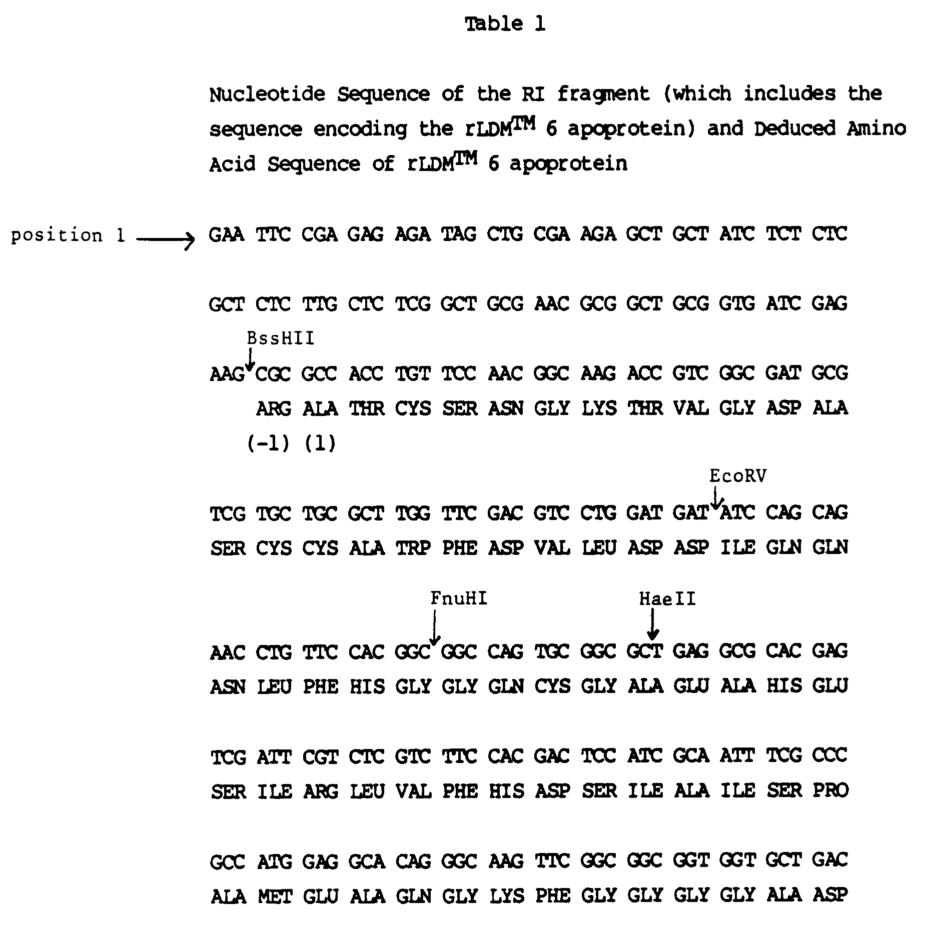

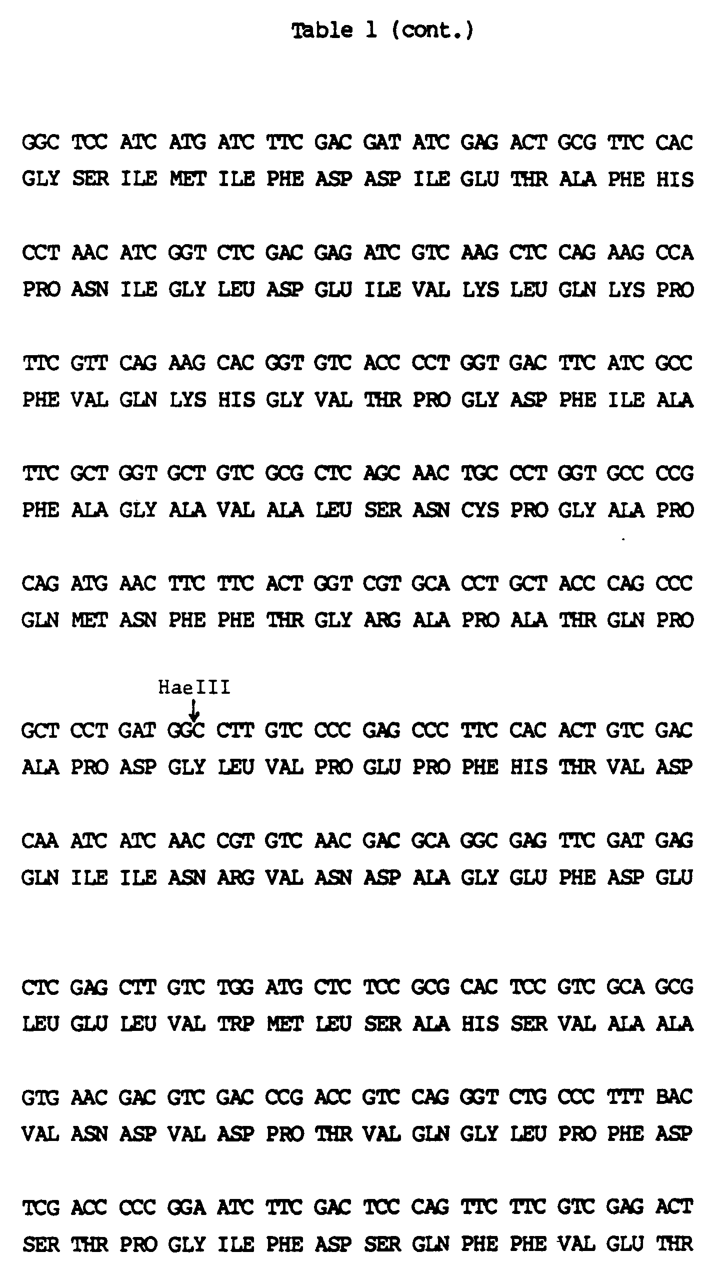

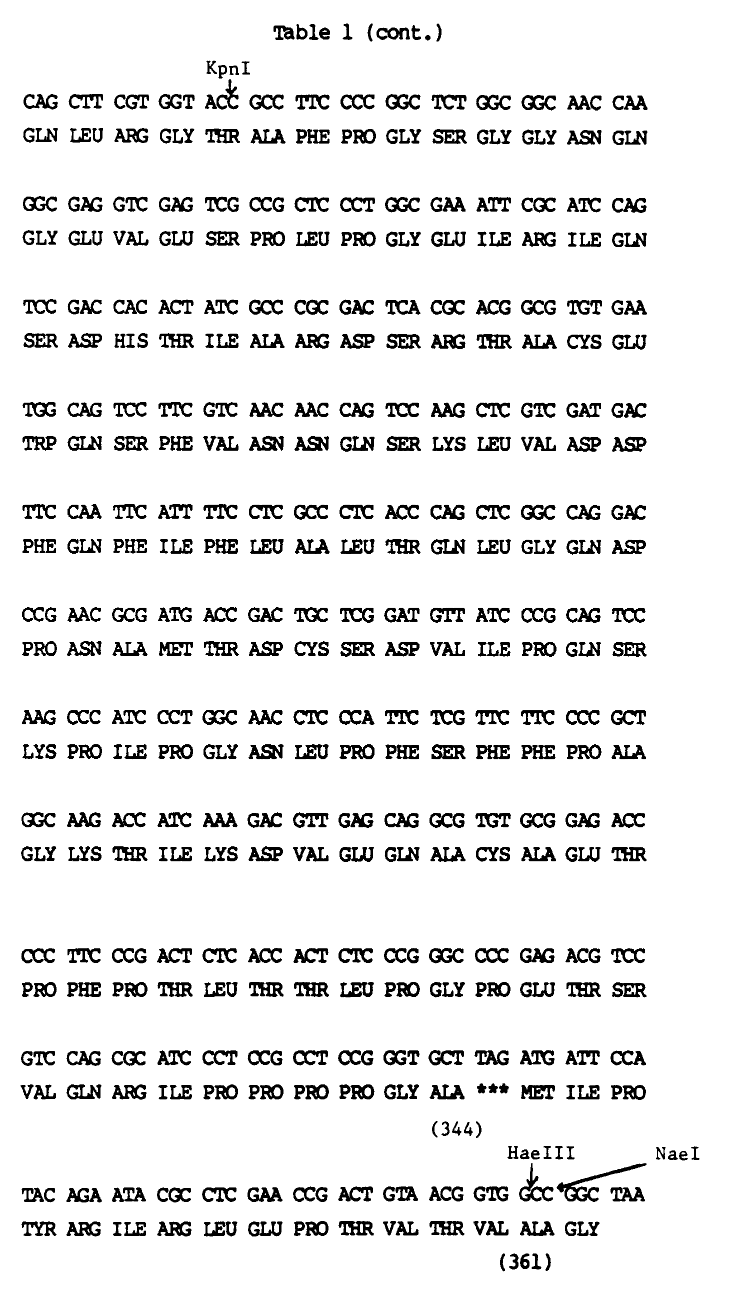

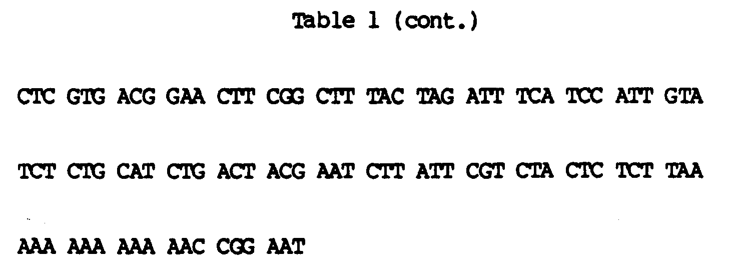

In Table 1 the DNA nucleotides of the coding sequence have been linearly grouped in triplets or codons consistent with the amino acid protein sequence for which the DNA encodes. The nucleotides have been numbered at various locations in Table 1 either above the line in which they appear or by the use of arrows. Table 1 also sets forth the amino acid protein sequence encoded for by the nucleotide sequence, and amino acids (and stops) in such protein sequence have been numbered in parenthesis below the line in which they appear (positive numbering beginning with the first amino acid of the ascertained mature sequence). Nucleotide triplets (codons) representing stop signals have been assigned a triple asterisk in the predicted amino acid sequence, it being understood that the codon TAG is not an efficient terminator in suppressing hosts; thus the protein is extended to the next TAA codon. In contrast, in suppressor-free hosts the protein will terminate at the TAG codon. The particular suppressor strain in which the protein is expressed will determine which amino acid is incoporated at the amber stop codon designated by the triple asterisk.

It has been ascertained that part of the nucleotide sequence prior to the 1-position Ala is an artifact or otherwise unrelated to the presumed signal sequence encoded by the native mRNA.

## Claims

1. DNA coding for a ligninase apoprotein comprising the following amino acid sequence:

                                                10
ALA THR CYS SER ASN GLY LYS THR VAL GLY ASP ALA
                        20
SER CYS CYS ALA TRP PHE ASP VAL LEU ASP ASP ILE GLN GLN
            30                                      40
ASN LEU PHE HIS GLY GLY GLN CYS GLY ALA GLU ALA HIS GLU
                                    50
SER ILE ARG LEU VAL PHE HIS ASP SER ILE ALA ILE SER PRO
                    60
ALA MET GLU ALA GLN GLY LYS PHE GLY GLY GLY GLY ALA ASP
    70                                  80
GLY SER ILE MET ILE PHE ASP ASP ILE GLU THR ALA PHE HIS
                            90
PRO ASN ILE GLY LEU ASP GLU ILE VAL LYS LEU GLN LYS PRO
            100                                     110
PHE VAL GLN LYS HIS GLY VAL THR PRO GLY ASP PHE ILE ALA
                                    120
PHE ALA GLY ALA VAL ALA LEU SER ASN CYS PRO GLY ALA PRO
                    130
GLN MET ASN PHE PHE THR GLY ARG ALA PRO ALA THR GLN PRO
    140                                 150
ALA PRO ASP GLY LEU VAL PRO GLU PRO PHE HIS THR VAL ASP
                            160
GLN ILE ILE ASN ARG VAL ASN ASP ALA GLY GLU PHE ASP GLU
            170                                     180
LEU GLU LEU VAL TRP MET LEU SER ALA HIS SER VAL ALA ALA
                                    190
VAL ASN ASP VAL ASP PRO THR VAL GLN GLY LEU PRO PHE ASP
                    200
SER THR PRO GLY ILE PHE ASP SER GLN PHE PHE VAL GLU THR
    210                                 220
GLN LEU ARG GLY THR ALA PHE PRO GLY SER GLY GLY ASN GLN
                            230
GLY GLU VAL GLU SER PRO LEU PRO GLY GLU ILE ARG ILE GLN
            240                                     250
SER ASP HIS THR ILE ALA ARG ASP SER ARG THR ALA CYS GLU
                                    260
TRP GLN SER PHE VAL ASN ASN GLN SER LYS LEU VAL ASP ASP
                    270
PHE GLN PHE ILE PHE LEU ALA LEU THR GLN LEU GLY GLN ASP
    280                                 290
PRO ASN ALA MET THR ASP CYS SER ASP VAL ILE PRO GLN SER

                                          300
LYS  PRO  ILE  PRO  GLY  ASN  LEU  PRO  PHE  SER  PHE  PHE  PRO  ALA
               310                                               320
GLY  LYS  THR  ILE  LYS  ASP  VAL  GLU  GLN  ALA  CYS  ALA  GLU  THR
                                             330
PRO  PHE  PRO  THR  LEU  THR  THR  LEU  PRO  GLY  PRO  GLU  THR  SER
                         340                 344
VAL  GLN  ARG  ILE  PRO  PRO  PRO  PRO  GLY  ALA.

2. A DNA sequence according to claim 1 the 5' end of which is operably linked to a signal sequence and/or control sequences for expression in a procaryotic and/or eucaryotic host.

3. A vector containing and capable of expressing the DNA sequence as defined in claims 1 or 2.

4. A plasmid selected from pBSR3, pLn104, pLn105, pLn106 and pLn1001 which have been deposited under Depository No. NRRL B-18068, 18067, 18156, 18157 and 18163 respectively.

5. Recombinant host cells transformed with the DNA sequence as defined in claims 1 or 2.

6. A method for producing a ligninase apoprotein which comprises culturing the transformed cells of claim 5.

7. A non-glycosylated ligninase apoprotein comprising the following amino acid sequence:

ALA THR CYS SER ASN GLY LYS THR VAL GLY ASP ALA
SER CYS CYS ALA TRP PHE ASP VAL LEU ASP ASP ILE GLN GLN
ASN LEU PHE HIS GLY GLY GLN CYS GLY ALA GLU ALA HIS GLU
SER ILE ARG LEU VAL PHE HIS ASP SER ILE ALA ILE SER PRO
ALA MET GLU ALA GLN GLY LYS PHE GLY GLY GLY GLY ALA ASP
GLY SER ILE MET ILE PHE ASP ASP ILE GLU THR ALA PHE HIS
PRO ASN ILE GLY LEU ASP GLU ILE VAL LYS LEU GLN LYS PRO
PHE VAL GLN LYS HIS GLY VAL THR PRO GLY ASP PHE ILE ALA
PHE ALA GLY ALA VAL ALA LEU SER ASN CYS PRO GLY ALA PRO
GLN MET ASN PHE PHE THR GLY ARG ALA PRO ALA THR GLN PRO
ALA PRO ASP GLY LEU VAL PRO GLU PRO PHE HIS THR VAL ASP
GLN ILE ILE ASN ARG VAL ASN ASP ALA GLY GLU PHE ASP GLU
LEU GLU LEU VAL TRP MET LEU SER ALA HIS SER VAL ALA ALA
VAL ASN ASP VAL ASP PRO THR VAL GLN GLY LEU PRO PHE ASP
SER THR PRO GLY ILE PHE ASP SER GLN PHE PHE VAL GLU THR
GLN LEU ARG GLY THR ALA PHE PRO GLY SER GLY GLY ASN GLN
GLY GLU VAL GLU SER PRO LEU PRO GLY GLU ILE ARG ILE GLN
SER ASP HIS THR ILE ALA ARG ASP SER ARG THR ALA CYS GLU
TRP GLN SER PHE VAL ASN ASN GLN SER LYS LEU VAL ASP ASP
PHE GLN PHE ILE PHE LEU ALA LEU THR GLN LEU GLY GLN ASP
PRO ASN ALA MET THR ASP CYS SER ASP VAL ILE PRO GLN SER
LYS PRO ILE PRO GLY ASN LEU PRO PHE SER PHE PHE PRO ALA
GLY LYS THR ILE LYS ASP VAL GLU GLN ALA CYS ALA GLU THR
PRO PHE PRO THR LEU THR THR LEU PRO GLY PRO GLU THR SER
VAL GLN ARG ILE PRO PRO PRO PRO GLY ALA.

8. A process for reconstituting an active ligninase enzyme which comprises the step of reacting a ligninase apoprotein, as defined in claim 7, in a reduced form with a heme in the presence of a sulfhydryl redox system.

9. A process according to claim 8 in which said redox system is glutathione.

## Patentansprüche

1. DNS, die für ein Ligninase Apoprotein codiert welches die nachfolgende Aminosäuresequenz enthält:

10
ALA THR CYS SER ASN GLY LYS THR VAL GLY ASP ALA
20
SER CYS CYS ALA TRP PHE ASP VAL LEU ASP ASP ILE GLN GLN
30 40
ASN LEU PHE HIS GLY GLY GLN CYS GLY ALA GLU ALA HIS GLU
50
SER ILE ARG LEU VAL PHE HIS ASP SER ILE ALA ILE SER PRO
60
ALA MET GLU ALA GLN GLY LYS PHE GLY GLY GLY GLY ALA ASP
70 80
GLY SER ILE MET ILE PHE ASP ASP ILE GLU THR ALA PHE HIS
90
PRO ASN ILE GLY LEU ASP GLU ILE VAL LYS LEU GLN LYS PRO
100 110
PHE VAL GLN LYS HIS GLY VAL THR PRO GLY ASP PHE ILE ALA
120
PHE ALA GLY ALA VAL ALA LEU SER ASN CYS PRO GLY ALA PRO
130
GLN MET ASN PHE PHE THR GLY ARG ALA PRO ALA THR GLN PRO
140 150
ALA PRO ASP GLY LEU VAL PRO GLU PRO PHE HIS THR VAL ASP
160
GLN ILE ILE ASN ARG VAL ASN ASP ALA GLY GLU PHE ASP GLU
170 180
LEU GLU LEU VAL TRP MET LEU SER ALA HIS SER VAL ALA ALA
190
VAL ASN ASP VAL ASP PRO THR VAL GLN GLY LEU PRO PHE ASP
200
SER THR PRO GLY ILE PHE ASP SER GLN PHE PHE VAL GLU THR
210 220
GLN LEU ARG GLY THR ALA PHE PRO GLY SER GLY GLY ASN GLN
230
GLY GLU VAL GLU SER PRO LEU PRO GLY GLU ILE ARG ILE GLN
240 250
SER ASP HIS THR ILE ALA ARG ASP SER ARG THR ALA CYS GLU
260
TRP GLN SER PHE VAL ASN ASN GLN SER LYS LEU VAL ASP ASP
270
PHE GLN PHE ILE PHE LEU ALA LEU THR GLN LEU GLY GLN ASP
280 290
PRO ASN ALA MET THR ASP CYS SER ASP VAL ILE PRO GLN SER
300
LYS PRO ILE PRO GLY ASN LEU PRO PHE SER PHE PHE PRO ALA
310 320
GLY LYS THR ILE LYS ASP VAL GLU GLN ALA CYS ALA GLU THR
330
PRO PHE PRO THR LEU THR THR LEU PRO GLY PRO GLU THR SER
340 344
VAL GLN ARG ILE PRO PRO PRO PRO GLY ALA.

2. Eine DNS Sequenz gemäss Anspruch 1, worin das 5'Ende operabel verbunden ist mit einer Signalsequenz und/oder Kontrolsequenz zur Expression in einem prokaryotischen und/oder eukaryotischen Wirt.

3. Ein Vektor enthaltend und geeignet zur Expression der DNS Sequenz wie in den Ansprüchen 1 und 2 definiert.

4. Ein Plasmid ausgewählt aus pBSR3, pLn104, pLn105, pLn106 und pLn1001 die mit den Hinterlegungsnummern NRRL B-18068, 18067, 18156, 18157 und 18163 hinterlegt wurden.

5. Rekombinante Wirtszellen transformiert mit der DNS Sequenz wie in den Ansprüchen 1 oder 2 definiert.

6. Eine Methode zur Herstellung eines Ligninase Apoproteins die die Züchtung der transformierten Zellen gemäss Anspruch 5 umfasst.

7. Ein nicht-glykosiliertes Ligninase Apoprotein welches die nachfolgende Aminosäuresequenz enthält:

ALA THR CYS SER ASN GLY LYS THR VAL GLY ASP ALA
SER CYS CYS ALA TRP PHE ASP VAL LEU ASP ASP ILE GLN GLN
ASN LEU PHE HIS GLY GLY GLN CYS GLY ALA GLU ALA HIS GLU
SER ILE ARG LEU VAL PHE HIS ASP SER ILE ALA ILE SER PRO
ALA MET GLU ALA GLN GLY LYS PHE GLY GLY GLY GLY ALA ASP
GLY SER ILE MET ILE PHE ASP ASP ILE GLU THR ALA PHE HIS
PRO ASN ILE GLY LEU ASP GLU ILE VAL LYS LEU GLN LYS PRO
PHE VAL GLN LYS HIS GLY VAL THR PRO GLY ASP PHE ILE ALA
PHE ALA GLY ALA VAL ALA LEU SER ASN CYS PRO GLY ALA PRO
GLN MET ASN PHE PHE THR GLY ARG ALA PRO ALA THR GLN PRO
ALA PRO ASP GLY LEU VAL PRO GLU PRO PHE HIS THR VAL ASP
GLN ILE ILE ASN ARG VAL ASN ASP ALA GLY GLU PHE ASP GLU
LEU GLU LEU VAL TRP MET LEU SER ALA HIS SER VAL ALA ALA
VAL ASN ASP VAL ASP PRO THR VAL GLN GLY LEU PRO PHE ASP
SER THR PRO GLY ILE PHE ASP SER GLN PHE PHE VAL GLU THR

GLN LEU ARG GLY THR ALA PHE PRO GLY SER GLY GLY ASN GLN
GLY GLU VAL GLU SER PRO LEU PRO GLY GLU ILE ARG ILE GLN
SER ASP HIS THR ILE ALA ARG ASP SER ARG THR ALA CYS GLU
TRP GLN SER PHE VAL ASN ASN GLN SER LYS LEU VAL ASP ASP
PHE GLN PHE ILE PHE LEU ALA LEU THR GLN LEU GLY GLN ASP
PRO ASN ALA MET THR ASP CYS SER ASP VAL ILE PRO GLN SER
LYS PRO ILE PRO GLY ASN LEU PRO PHE SER PHE PHE PRO ALA
GLY LYS THR ILE LYS ASP VAL GLU GLN ALA CYS ALA GLU THR
PRO PHE PRO THR LEU THR THR LEU PRO GLY PRO GLU THR SER
VAL GLN ARG ILE PRO PRO PRO PRO GLY ALA.

8. Ein Verfahren zur Wiederherstellung eines aktiven Ligninase - Enzyms das die Stufe der Umsetzung eines Ligninase Apoproteins, das im Anspruch 7 definiert wird, in reduzierter Form mit einem Häm in Gegenwart eines Sulfhydryl Redoxsystems umfasst.

9. Ein Verfahren gemäss Anspruch 8 worin das Redoxsystem Glutathion ist.

## Revendications

1. ADN codant pour une apoprotéine de ligninase, comprenant la séquence d'aminoacides suivante:

10
ALA THR CYS SER ASN GLY LYS THR VAL GLY ASP ALA
20
SER CYS CYS ALA TRP PHE ASP VAL LEU ASP ASP ILE GLN GLN
30
ASN LEU PHE HIS GLY GLY GLN CYS GLY ALA GLU ALA HIS GLU
40
50
SER ILE ARG LEU VAL PHE HIS ASP SER ILE ALA ILE SER PRO
60
ALA MET GLU ALA GLN GLY LYS PHE GLY GLY GLY GLY ALA ASP
70
80
GLY SER ILE MET ILE PHE ASP ASP ILE GLU THR ALA PHE HIS
90
PRO ASN ILE GLY LEU ASP GLU ILE VAL LYS LEU GLN LYS PRO
100
110
PHE VAL GLN LYS HIS GLY VAL THR PRO GLY ASP PHE ILE ALA
120
PHE ALA GLY ALA VAL ALA LEU SER ASN CYS PRO GLY ALA PRO
130
GLN MET ASN PHE PHE THR GLY ARG ALA PRO ALA THR GLN PRO
140
150
ALA PRO ASP GLY LEU VAL PRO GLU PRO PHE HIS THR VAL ASP
160
GLN ILE ILE ASN ARG VAL ASN ASP ALA GLY GLU PHE ASP GLU
170
180
LEU GLU LEU VAL TRP MET LEU SER ALA HIS SER VAL ALA ALA
190
VAL ASN ASP VAL ASP PRO THR VAL GLN GLY LEU PRO PHE ASP
200
SER THR PRO GLY ILE PHE ASP SER GLN PHE PHE VAL GLU THR
210
220
GLN LEU ARG GLY THR ALA PHE PRO GLY SER GLY GLY ASN GLN
230
GLY GLU VAL GLU SER PRO LEU PRO GLY GLU ILE ARG ILE GLN
240
250
SER ASP HIS THR ILE ALA ARG ASP SER ARG THR ALA CYS GLU
260
TRP GLN SER PHE VAL ASN ASN GLN SER LYS LEU VAL ASP ASP
270
PHE GLN PHE ILE PHE LEU ALA LEU THR GLN LEU GLY GLN ASP
280
290
PRO ASN ALA MET THR ASP CYS SER ASP VAL ILE PRO GLN SER

                                        300
Lys Pro Ile Pro Gly Asn Leu Pro Phe Ser Phe Phe Pro Ala
                310                                                                    320
Gly Lys Thr Ile Lys Asp Val Glu Gln Ala Cys Ala Glu Thr
                                                            330
Pro Phe Pro Thr Leu Thr Thr Leu Pro Gly Pro Glu Thr Ser
                                340                         344
Val Gln Arg Ile Pro Pro Pro Pro Gly Ala.

2. Séquence d'ADN selon la revendication 1, dont l'extrémité 5' est fonctionnellement liée à une séquence signal et/ou des séquences pour le contrôle de son expression dans un hôte eucaryote et/ou un hôte procaryote.

3. Vecteur contenant et capable d'exprimer la séquence d'ADN telle que définie dans la revendication 1 ou 2.

4. Plamide choisi parmi pBSR3, pLn104, pLn105, pLn106 et pLn1001 qui ont été déposés sous les n° NRRL B-18068, 18067, 18156, 18157 et 18163, respectivement.

5. Cellules hôtes recombinantes transformées par la séquence d'ADN telle que définie dans la revendication 1 ou 2.

6. Procédé pour la production d'une apoprotéine de ligninase, qui comprend la culture des cellules transformées de la revendication 5.

7. Apoprotéine ligninase non glycosylée, comprenant la séquence d'aminoacides suivante:

ALA THR CYS SER ASN GLY LYS THR VAL GLY ASP ALA
SER CYS CYS ALA TRP PHE ASP VAL LEU ASP ASP ILE GLN GLN
ASN LEU PHE HIS GLY GLY GLN CYS GLY ALA GLU ALA HIS GLU
SER ILE ARG LEU VAL PHE HIS ASP SER ILE ALA ILE SER PRO
ALA MET GLU ALA GLN GLY LYS PHE GLY GLY GLY GLY ALA ASP
GLY SER ILE MET ILE PHE ASP ASP ILE GLU THR ALA PHE HIS
PRO ASN ILE GLY LEU ASP GLU ILE VAL LYS LEU GLN LYS PRO
PHE VAL GLN LYS HIS GLY VAL THR PRO GLY ASP PHE ILE ALA
PHE ALA GLY ALA VAL ALA LEU SER ASN CYS PRO GLY ALA PRO
GLN MET ASN PHE PHE THR GLY ARG ALA PRO ALA THR GLN PRO
ALA PRO ASP GLY LEU VAL PRO GLU PRO PHE HIS THR VAL ASP
GLN ILE ILE ASN ARG VAL ASN ASP ALA GLY GLU PHE ASP GLU
LEU GLU LEU VAL TRP MET LEU SER ALA HIS SER VAL ALA ALA
VAL ASN ASP VAL ASP PRO THR VAL GLN GLY LEU PRO PHE ASP
SER THR PRO GLY ILE PHE ASP SER GLN PHE PHE VAL GLU THR
GLN LEU ARG GLY THR ALA PHE PRO GLY SER GLY GLY ASN GLN
GLY GLU VAL GLU SER PRO LEU PRO GLY GLU ILE ARG ILE GLN
SER ASP HIS THR ILE ALA ARG ASP SER ARG THR ALA CYS GLU
TRP GLN SER PHE VAL ASN ASN GLN SER LYS LEU VAL ASP ASP
PHE GLN PHE ILE PHE LEU ALA LEU THR GLN LEU GLY GLN ASP
PRO ASN ALA MET THR ASP CYS SER ASP VAL ILE PRO GLN SER
LYS PRO ILE PRO GLY ASN LEU PRO PHE SER PHE PHE PRO ALA
GLY LYS THR ILE LYS ASP VAL GLU GLN ALA CYS ALA GLU THR
PRO PHE PRO THR LEU THR THR LEU PRO GLY PRO GLU THR SER
VAL GLN ARG ILE PRO PRO PRO PRO GLY ALA.

8. Procédé pour la reconstitution d'une enzyme de type ligninase active, comprenant les étapes de mise en réaction d'une apoprotéine de ligninase, telle que définie dans la revendication 7, sous une forme réduite, avec un hème, en présence d'un système rédox sulfhydryle.

9. Procédé selon la revendication 8, dans lequel ledit système rédox est le glutathion.
